Europäisches Patentamt

European Patent Office

Office européen des brevets

(19)

(11) **EP 1 019 191 B1**

(12) **FASCICULE DE BREVET EUROPEEN**

(45) Date de publication et mention
de la délivrance du brevet:
**12.03.2003 Bulletin 2003/11**

(21) Numéro de dépôt: **98943986.4**

(22) Date de dépôt: **15.09.1998**

(51) Int Cl.⁷: **B01J 35/00**, B01J 37/03

(86) Numéro de dépôt international:
**PCT/FR98/01967**

(87) Numéro de publication internationale:
**WO 99/015271 (01.04.1999 Gazette 1999/13)**

(54) **PARTICULES POLYMETALLIQUES ULTRAFINES, LEUR PREPARATION ET LEUR UTILISATION POUR L'HYDROGENATION D'OLEFINES OU POUR LE COUPLAGE DE DERIVES HALOGENES AROMATIQUES**

POLYMETALLISCHES ULTRAFEINES PARTIKEL, IHRE BEREITUNG UND IHRE VERWENDUNG FÜR DIE HYDRIERUNG VON OLEFINEN ODER FÜR DIE KUPPLUNG VON HALOGENIERTE DERIVATEN

ULTRAFINE POLYMETALLIC PARTICLES, PREPARATION AND USE FOR HYDROGENATING OLEFINS AND FOR COUPLING HALOGENATED AROMATIC DERIVATIVES

(84) Etats contractants désignés:
**AT BE CH CY DE DK ES FI FR GB GR IE IT LI LU MC NL PT SE**

(30) Priorité: **23.09.1997 FR 9711814**

(43) Date de publication de la demande:
**19.07.2000 Bulletin 2000/29**

(73) Titulaire: **CENTRE NATIONAL DE LA RECHERCHE SCIENTIFIQUE (CNRS)**
**75016 Paris (FR)**

(72) Inventeurs:
• **DUBOIS, Jean-Marie**
**F-54340 Pompey (FR)**

• **FORT, Yves**
**F-54500 Vandoeuvre les Nancy (FR)**
• **TILLEMENT, Olivier**
**F-54160 Froloy (FR)**

(74) Mandataire: **Sueur, Yvette et al**
**Cabinet SUEUR & L'HELGOUALCH,**
**109, boulevard Haussmann**
**75008 Paris (FR)**

(56) Documents cités:
**EP-A- 0 522 669**       **WO-A-97/33690**
**DE-A- 4 443 705**       **US-A- 5 147 841**

• **Physikalische Chemie, Peter W. Atkins, VCH Verlag Weinheim, 1987, page 868.**

**Description**

La présente invention a pour objet un procédé de préparation de particules polymétalliques ultrafines, les particules obtenues, et l'utilisation des particules obtenues, notamment pour la catalyse.

Les particules métalliques fines sont connues pour diverses applications, notamment en catalyse. Différents procédés pour les préparer ont été décrits.

L. K. Kurihara, et al, (Nanostructured Materials, vol. 5, n° 6, 607-613, 1995) décrit la préparation de particules nanométriques d'un métal par un procédé dit "polyol", mis en oeuvre pour divers métaux faciles à réduire tels que par exemple Fe, Co, Ni, Cu, Ru, Rh, Pd, Ag, Sn, Re, W, Pt, seuls ou en mélange. Un tel procédé consiste à dissoudre ou à mettre en suspension un précurseur du dit métal dans un polyol, puis à chauffer au reflux pour obtenir un précipité de particules métalliques. Les particules obtenues peuvent être nanométriques. Elles sont polymétalliques lorsque le milieu réactionnel contient des sels de métaux différents. L'utilisation d'un sel précurseur d'aluminium ou d'un autre métal difficile à réduire n'est pas mentionnée. Pour les métaux autres que les métaux nobles, les dimensions des cristallites des particules sont supérieures ou égales à 8 nm, plus généralement entre 10 et 80 nm. Seul Ru et Pt permettent d'obtenir des dimensions moyennes de cristallites plus faibles, respectivement de 5 et 2 nm. L'obtention de particules nanométriques de Cu-Co est décrite dans L. K. Kurihara, et al, J. Mat. Res. Vol. 10, n° 6, juin 1995. Dans ce cas, il s'agit de deux métaux ayant un potentiel standard d'oxydation entre -1,20 V et +0,8 V, c'est-à-dire de métaux faciles à réduire. Le procédé dit "polyol" ne permet cependant pas la préparation de particules fines d'un métal difficile à réduire tel que l'aluminium par exemple.

Des particules nanométriques contenant de l'aluminium peuvent être préparées en réduisant un sel par LiAlH$_4$. Ainsi la préparation de particules nanocristallines Ti/Al et Ni/Ai à partir d'un chlorure du métal correspondant en présence de LiAlH$_4$ est décrite par J.A. Haber, et al, (Advanced Materials, 1996, 8 n° 2). Le mécanisme réactionnel de ce procédé étant le suivant :

$$M^{n+} + nLiAlH_4 \rightarrow nLi^+ + M + nAlH_3 + n/2\ H_2\nearrow$$

$$nAlH_3 \rightarrow 3n/2\ H_2\nearrow + A\ell$$

la teneur en Al présente une limite supérieure correspondant à la valence du métal associé. En outre, la structure des poudres obtenues est difficilement contrôlable, la cinétique de décomposition de AlH$_3$ étant peu prévisible.

On connaît en outre la préparation de particules intermétalliques nanométriques à l'aide de borohydrures. S. T. Schwab, et al, (Materials Science and Engineering A204 (1995) 197-200), décrivent la préparation de particules intermétalliques par réduction d'un mélange de sels de différents métaux par un borohydrure. (par exemple TiCl$_4$ + AlCl$_3$ + triéthylborohydrure de lithium). Ce procédé permet d'obtenir des particules intermétalliques sans limitation sur les teneurs en métaux respectifs. Il requiert néanmoins un traitement thermique à 1000°C pour décomposer les produits obtenus par réaction des sels avec le borohydrure et obtenir les formes métalliques.

D'autres procédés sont connus, consistant à réduire des sels de métal soit par un borohydrure, soit par un hydrure alcalin activé par addition de BF$_3$. Dans tous les cas, la présence de bore dans le milieu réactionnel introduit un risque de pollution.

On connaît également la préparation de particules fines monométalliques par réaction d'un sel de métal avec l'hydrure de sodium en solution dans un solvant organique et en présence d'un alcoolate. Un tel procédé a été décrit pour des particules de nickel et pour des particules de zinc (Paul Caubère, et al, J. Am. Chem. Soc., 1982, 104, 7130 ; P. Gallezot, C. Leclercq, Y. Fort, P. Caubère, Journal of Molecular Catalysis, 93 (1994) 79 83, pp. 80-83 ; Brunet, et al, Journal of Organic Chemistry 1980, vol. 45, pp. 1937-1945). Les particules obtenues ont une dimension de cristallites de l'ordre du nm et sont utiles notamment comme catalyseur pour des catalyses hétérogènes. Lorsqu'elles sont préparées en présence d'un ligand phosphine ou 2,2'-bipyridine, elles perdent leurs propriétés réductrices et se comportent comme un agent de couplage (Lourak, et al, Journal of Organic Chemistry 1989, vol. 54, pp. 4840-4844).

Un procédé analogue a également été décrit pour la préparation de particules monométalliques de Pd, qui peuvent être utilisées comme catalyseur d'hydrogénation d'acétylène. (J.J. Brunet et P. Caubère, J. Org. Chem. 1984, 49, 4058-4060).

Ce procédé est particulièrement intéressant, mais ne peut être efficacement utilisé pour préparer des particules d'un métal difficile à réduire, ayant un potentiel d'oxydation standard à 25°C inférieur à -1,20 V, tel que l'aluminium par exemple. De plus, la réduction d'un cation Al$^{3+}$ en présence d'hydrure de sodium ou de lithium n'a jamais été évoquée dans ces procédés.

On connaît l'utilité des particules ultrafines polymétalliques comme catalyseur dans différentes réactions, notamment celles qui contiennent de l'aluminium ou un autre métal dont les sels sont difficiles à réduire. Or les procédés de l'art antérieur ne permettent pas d'obtenir aisément de particules polymétalliques ultrafines contenant de l'aluminium ayant une bonne pureté, avec une teneur en aluminium quelconque. Le but de la présente invention est de proposer un tel procédé.

Les présents inventeurs ont maintenant trouvé que, bien que la réduction d'un sel d'aluminium par l'hydrure de lithium ou de sodium donne des particules ultrafines d'aluminium métallique qui se réoxydent rapidement dans le milieu réactionnel, il était possible d'obtenir des particules polymétalliques stables contenant l'aluminium et un autre métal, la teneur en Al étant quelconque, par réduction d'un mélange de sels par un hydrure de métal alcalin ou alcalino-terreux.

La présente invention a ainsi pour objet un procédé de préparation de particules polymétalliques nanométriques, les particules obtenues, ainsi que leurs utilisations.

Le procédé de la présente invention est un procédé de préparation de particules polymétalliques nanométriques par réduction d'un mélange de sels en solution dans un solvant organique à l'aide d'un hydrure de métal alcalin ou alcalino-terreux, à une température inférieure ou égale à la température de reflux du solvant, l'un au moins des sels étant un sel d'un métal ayant un potentiel d'oxydation standard $E^o_{Mn+/M}$ à 25°C supérieur à -1,18 V. Il est caractérisé en ce que le mélange de sels en solution comprend au moins un sel d'un métal ayant un potentiel d'oxydation standard $E^o_{Mn+/M}$ à 25°C inférieur à -1,20 V.

Le procédé est mis en oeuvre avantageusement en ajoutant au milieu réactionnel, un alcoolate aliphatique ou aromatique de métal alcalin, de métal alcalino-terreux ou de métal de transition ou un aminoalcoolate de métal alcalin, de métal alcalino-terreux ou de métal de transition. L'addition d'un tel alcoolate a pour effet d'activer la réaction de réduction des ions métalliques par l'hydrure.

Les sels utilisés dans le procédé de la présente invention peuvent être choisis parmi les halogénures. Ils peuvent également être choisis parmi les sels organiques tel que les acétates, les acétylacétonates ou parmi les alcoolates.

Le sel d'un métal ayant un potentiel d'oxydation standard $E^o_{Mn+/M}$ à 25°C inférieur à -1,20 V, (c'est-à-dire le sel d'un métal difficile à réduire) est un sel d'un métal choisi dans le groupe constitué par Zr, Ce, Ti, Hf et Al. Les sels d'aluminium sont particulièrement intéressants.

Le sel d'un métal ayant un potentiel d'oxydation standard $E^o_{Mn+/M}$ à 25°C supérieur à -1,18 V, (c'est-à-dire le sel d'un métal plus facile à réduire) est un sel d'un métal choisi dans le groupe constitué par Ni, Co, Fe, Cu, Zn, Cd, Cr, Mn, Pd, Ag, Pt, Au, Bi, Sb. Parmi ces métaux, Pd, Ag, Pt et Au, qui ont un potentiel d'oxydation standard $E^o_{Mn+/M}$ à 25°C supérieur à +0,8 V, sont très faciles à réduire.

Les quantités relatives des divers sels utilisés pour la réaction de réduction sont choisies en fonction de la composition souhaitée pour les particules polymétalliques finales, étant entendu que les proportions sont conservées lors de la réaction de réduction.

L'agent réducteur utilisé dans le procédé de l'invention est un hydrure de métal alcalin ou alcalino-terreux. Parmi ces hydrures, on peut citer : LiH, NaH, KH, CaH$_2$ et MgH$_2$. LiH et NaH sont particulièrement préférés.

NaH peut avantageusement être utilisé sous la forme d'une solution commerciale à 65% en masse dans l'huile minérale, éventuellement après lavage par un solvant organique aprotique.

Comme hydrure de lithium, on peut utiliser un hydrure de lithium du commerce sous forme de poudre.

L'hydrure peut être activé par la présence d'un alcoolate ou d'un aminoalcoolate. Le tertiobutylate de sodium, le tertiobutylate de lithium, l'isopropylate d'aluminium et le N,N-diméthylaminoéthanolate de sodium sont particulièrement préférés. De préférence, l'alcoolate est formé in situ par action de l'hydrure sur un alcool aliphatique ou aromatique choisi de préférence dans le groupe constitué par le tertiobutanol, le méthyl-2-pentanol-2, le phénol, le monoéthyl étherdiéthylène glycol, le néopentanol, le cyclohexanol, l'éthanol, l'isopropanol.

La quantité d'hydrure (en nombre de moles) qui est ajoutée au milieu doit être au moins égale à la somme des valences des cations métalliques à réduire. Lorsque l'on souhaite activer la réduction par un alcoolate préparé in situ, il est nécessaire d'augmenter la quantité initiale d'hydrure de la quantité correspondant au moins à la stoechiométrie de la réaction de formation de l'alcoolate. Un excès d'hydrure est souhaitable, de préférence de l'ordre de 20%.

La réduction du sel de métal par l'hydrure alcalin ou alcalino-terreux est effectuée dans un solvant organique aprotique. Le solvant peut être choisi parmi les éthers aprotiques, les hydrocarbures aliphatiques, les hydrocarbures aromatiques. Un mélange de plusieurs solvants peut être utilisé. Les solvants suivants sont cités à titre d'exemple : tétrahydrofuranne, 1,2-diméthoxyéthane, anisole, hexane heptane, octane, benzène, toluène, xylène, mésitylène. Il est préférable d'utiliser des solvants préalablement distillés, purifiés et séchés, exempts de peroxydes.

Le procédé de la présente invention peut également être mis en oeuvre en présence d'un composé comprenant un ligand azoté ou phosphoré. Parmi les ligands azotés, on peut citer les bipyridines (par exemple la 2,2'-bipyridine, les dialkylpyridines et les dialkoxypyridines), et les terpyridines (par exemple la 2,2',6,6'-terpyridine).

Parmi les ligands phosphorés, on peut citer la triphénylphosphine, le 1,2-bis(diphénylphosphino)éthane, les alkylphosphines et les arylphosphines.

Pour la mise en oeuvre du procédé, les réactifs sont dispersés dans le solvant organique. Dans un mode de

**EP 1 019 191 B1**

réalisation du procédé, tous les réactifs sont introduits simultanément dans le réacteur. Dans un autre mode de réalisation, les réactifs sont introduits successivement dans le réacteur, l'ordre d'introduction n'étant pas critique.

La réaction de réduction du mélange de sels peut être effectuée à toute température comprise dans l'intervalle limité par la température de solidification et par la température de reflux du solvant utilisé. Il est particulièrement intéressant d'effectuer la réaction à la température de reflux du solvant lorsque le milieu réactionnel contient un sel d'un métal ayant un faible potentiel de réduction.

L'évolution de la réaction peut être suivie par la mesure de la quantité d'hydrogène dégagée, due à la réduction des sels métalliques et le cas échéant à la formation in situ des alcoolates destinés à activer l'hydrure.

Le matériau obtenu par le procédé proposé est constitué de particules nanométriques polymétalliques ayant une dimension moyenne de cristallites inférieure à 4 nm et une association intime des divers métaux présents à l'intérieur des grains, constituées d'au moins un métal choisi dans le groupe constitué par Ni, Co, Fe, Cu, Zn, Cd, Cr, Mn, Pd, Ag, Pt, Au, Bi et Sb, et d'au moins un métal choisi dans le groupe constitué par Zr, Ce, Ti, Hf et Al. Un matériau constitué de particules contenant au moins 70 % en nombre d'atomes d'Al est particulièrement préféré.

Les particules obtenues à la fin de la réaction de réduction peuvent être utilisées directement dans le solvant dans lequel elles ont été obtenues. On peut également éliminer ledit solvant pour obtenir des particules sous forme de poudre. Lorsqu'un traitement thermique est souhaité, il peut être effectué soit avant l'élimination du solvant, soit sur les poudres obtenues après élimination du solvant.

Les particules de la présente invention présentent des propriétes catalytiques dans de nombreuses réactions. Elles sont particulièrement efficaces comme catalyseur d'hydrogénation et comme catalyseur de couplage direct de dérivés halogénés aromatiques. Leur rendement et leur vitesse de réaction sont nettement supérieurs à ceux des particules monométalliques utilisées jusqu'ici. De manière surprenante, on a constaté que l'addition d'Al, qui n'a pas d'activité catalytique, à un métal ayant une activité catalytique intrinsèque, augmente l'activité catalytique globale.

La présente invention est illustrée ci-après par des exemples dans lesquels les performances catalytiques de particules polymétalliques obtenues par le procédé selon l'invention, sont comparées à celles de particules monométalliques obtenues par un procédé similaire.

Sur les figures jointes :

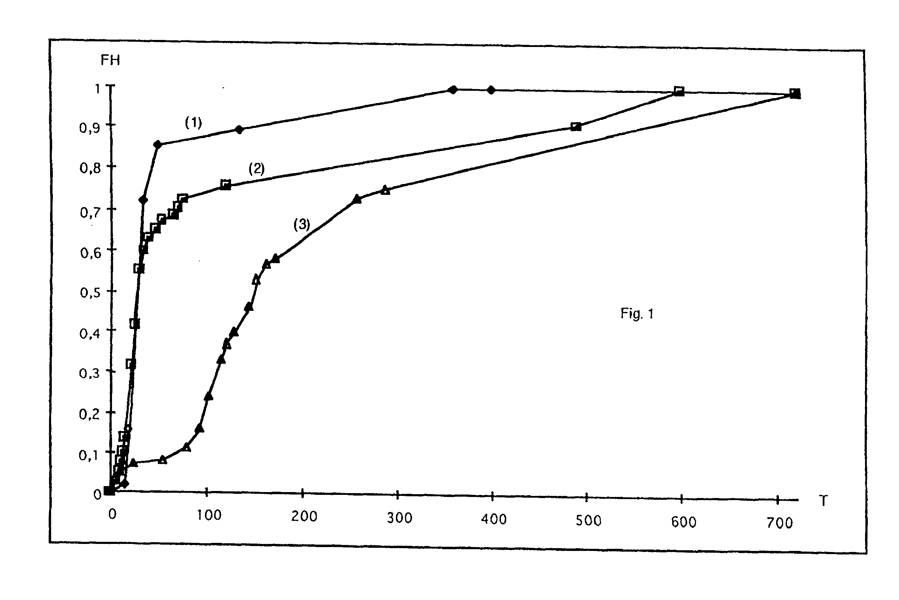

⇒ la figure 1 représente la fraction volumique FH d'hydrogène dégagé en fonction du temps T (exprimé en minutes), pour la réaction de l'exemple 1 (courbe 1), de l'exemple 2 (courbe 2) et de l'exemple 3 (courbe 3).

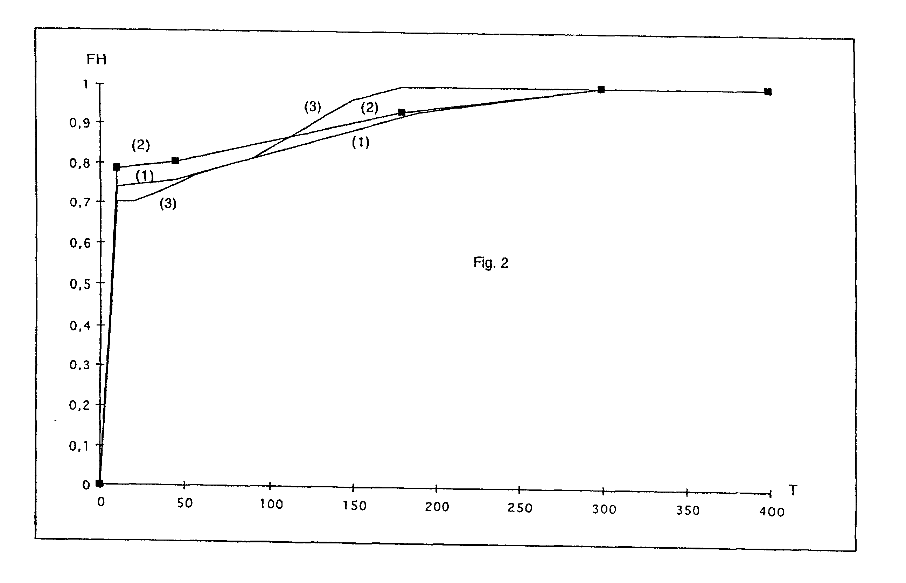

⇒ la figure 2 représente la fraction volumique FH d'hydrogène dégagé en fonction du temps T (exprimé en minutes), pour la réaction de l'exemple 4 (courbe 1), de l'exemple 5 (courbe 2) et de l'exemple 6 (courbe 3).

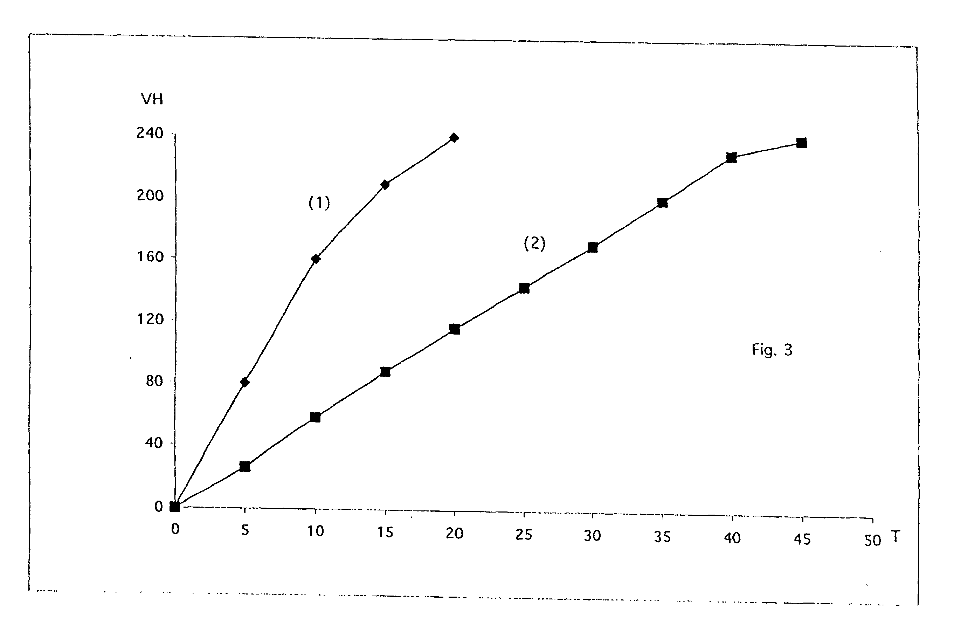

⇒ la figure 3 représente la courbe montrant la variation du volume d'hydrogène consommé VH (exprimé en ml) en fonction de la durée T (exprimée en minutes) au cours d'une réaction d'hydrogénation de styrène catalysée par des particules AlNi (courbe 1) et Ni (courbe 2).

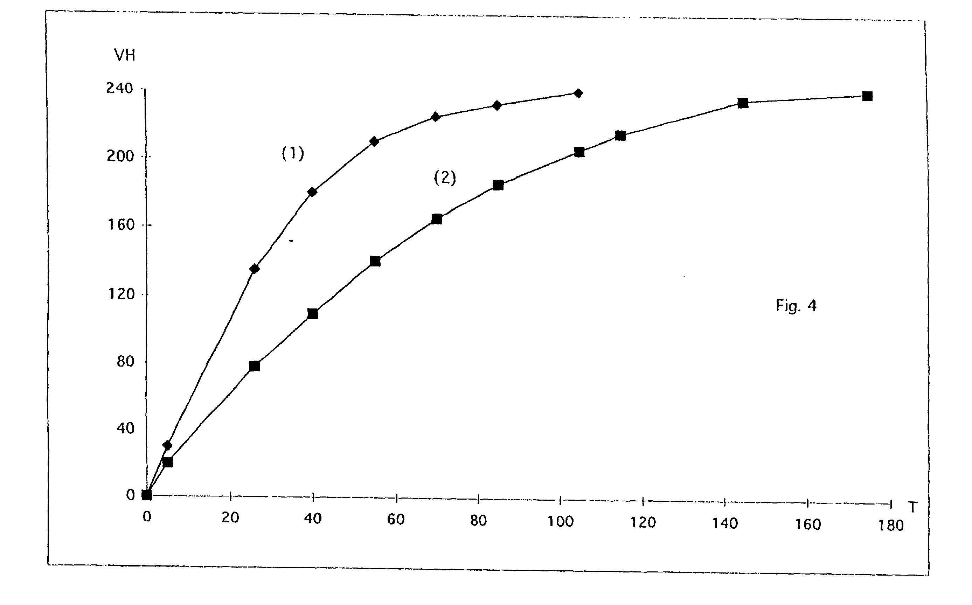

⇒ la figure 4 représente la courbe montrant la variation du volume d'hydrogène consommé VH (exprimé en ml) en fonction de la durée T (exprimée en minutes) au cours d'une réaction d'hydrogénation de cyclooctène catalysée par des particules AlNi (courbe 1) et Ni (courbe 2).

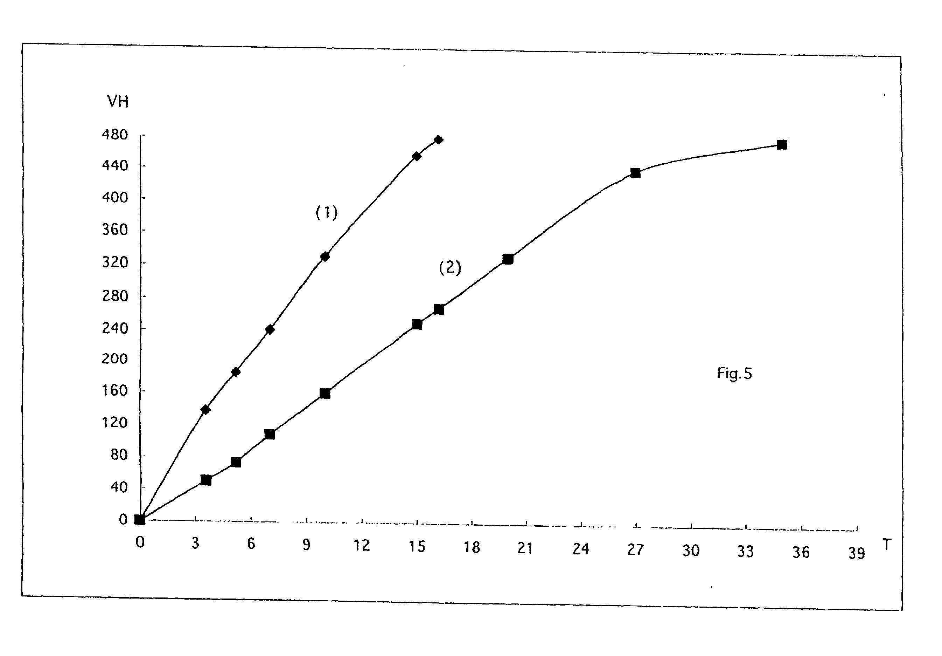

⇒ la figure 5 représente la courbe montrant la variation du volume d'hydrogène consommé VH (exprimé en ml) en fonction de la durée T (exprimée en minutes) au cours d'une réaction d'hydrogénation de diphénylacétylène catalysée par des particules AlNi (courbe 1) et Ni (courbe 2).

**Exemple 1**

Préparation de particules bimétalliques Al-Ni [1/1]

Dans un réacteur muni d'un réfrigérant, d'une ampoule de coulée et d'un thermomètre, on a introduit 0,72 g (90 mmoles) de LiH, 1,76 g (10 mmoles) d'acétate de nickel $Ni(OAc)_2$ et 3,24 g (10 mmoles) d'acétylacétonate d'aluminium $Al(Acac)_3$ en suspension dans 30 ml de THF à une température d'environ 20°C et sous atmosphère d'azote. La suspension obtenue a été portée à reflux (environ 63°C).

On a ensuite ajouté goutte à goutte en 10 min environ, par l'intermédiaire de l'ampoule de coulée, 1,48 g (20 mmoles) de t-BuOH en solution dans 10 ml de THF. La suspension a ensuite été agitée pendant 6 heures à 63°C.

La formation des particules métalliques a été suivie par la mesure du dégagement d'hydrogène résultant de la réaction de formation de t-BuOLi et de la réduction des sels métalliques. L'évolution du dégagement d'hydrogène est représentée sur la courbe 1 de la figure 1.

Après le chauffage de 6 heures à 63°C, les particules sont complètement formées et se présentent sous la forme d'une suspension de couleur noire.

4

**Exemple 2**

Préparation de particules bimétalliques Al-Ni [2/1]

On a opéré de la même manière que dans l'exemple 1, mais en utilisant 0,96 g (120 mmoles) de LiH, 1,76 g (10 mmoles) de Ni(OAc)$_2$ et 6,48 g (20 mmoles) de Al(Acac)$_3$.
La durée du chauffage à 63°C sous agitation était de 10 heures.
L'évolution du dégagement d'hydrogène est représentée sur la courbe 2 de la figure 1.

**Exemple 3**

Préparation de particules trimétalliques Al-Ni-Co [1/1/1]

On a opéré de la même manière que dans l'exemple 1, mais en utilisant 0,88 g (110 mmoles) de LiH, 1,76 g (10 mmoles) de Ni(OAc)$_2$, 1,76 g (10 mmoles) de Co(OAc)$_2$ et 3,24 g (10 mmoles) de Al(Acac)$_3$.
La durée du chauffage à 63°C sous agitation était de 12 heures.
L'évolution du dégagement d'hydrogène est représentée sur la courbe 3 de la figure 1.

**Exemple 4**

Préparation de particules bimétalliques Al-Ni [1/1]

On a opéré de la même manière que dans l'exemple 1, mais en utilisant 2,16 g (90 mmoles) de NaH, 1,76 g (10 mmoles) de Ni(OAc)$_2$ et 3,24 g (10 mmoles) de Al(Acac)$_3$.
La durée du chauffage à 63°C sous agitation était de 5 heures.
L'évolution du dégagement d'hydrogène est représentée sur la courbe 1 de la figure 2.

**Exemple 5**

Préparation de particules bimétalliques Al-Ni [1/1]

On a opéré de la même manière que dans l'exemple 1, mais en utilisant 2,16 g (90 mmoles) de NaH, 1,28 g (10 mmoles) de NiCl$_2$ et 3,24 g (10 mmoles) de Al(Acac)$_3$.
La durée du chauffage à 63°C sous agitation était de 6 heures
L'évolution du dégagement d'hydrogène est représentée sur la courbe 2 de la figure 2.

**Exemple 6**

Préparation de particules bimétalliques Al-Ni [1/1]

On a opéré de la même manière que dans l'exemple 1, mais en utilisant 2,16 g (90 mmoles) de NaH, 2,57 g (10 mmoles) de Ni(Acac)$_2$ et 3,24 g (10 mmoles) de Al(Acac)$_3$.
La durée du chauffage à 63°C sous agitation était de 5 heures.
L'évolution du dégagement d'hydrogène est représentée sur la courbe 3 de la figure 2.

**Exemple 7**

Préparation de particules bimétalliques Al-Ni [1/1]

On a opéré de la même manière que dans l'exemple 1, mais en utilisant 2,16 g (90 mmoles) de NaH, 2,57 g (10 mmoles) de Ni(Acac)$_2$ et 1,33 g (10 mmoles) de AlCl$_3$.
La durée du chauffage à 63°C sous agitation était de 5 heures.

**Exemple 8**

Préparation de particules bimétalliques Al-Ni [1/1]

On a opéré de la même manière que dans l'exemple 6, mais en utilisant 1,92 g (80 mmoles) de NaH, 2,57 g (10 mmoles) de Ni(Acac)$_2$, 3,24 g (10 mmoles) de Al(Acac)$_3$ et 0,74 g (10 mmoles) de t-BuOH.

La durée du chauffage à 63°C sous agitation était de 3 heures.

**Exemple 9**

Préparation de particules bimétalliques Al-Ni [1/1]

On a opéré de la même manière que dans l'exemple 6, mais en utilisant 2,28 g (95 mmoles) de NaH, 2/57 g (10 mmoles) de Ni(Acac)$_2$, 3,24 g (10 mmoles) de Al(Acac)$_3$ et 1,85 g (25 mmoles) de t-BuOH.

La durée du chauffage à 63°C sous agitation était de 2 heures.

**Exemple 10**

Préparation de particules bimétalliques Al-Ni [1/1]

On a opéré de la même manière que dans l'exemple 4, mais en ajoutant 3,12 g (20 mmoles) de 2,2'-bipyridine à la suspension initiale d'hydrure et de sels métalliques.

La durée du chauffage à 63°C sous agitation était de 3 heures.

Les conditions particulières de chacun des exemples ci-dessus sont rassemblées dans le tableau 1 suivant :

Tableau 1

| Ex | Précurseurs | mmol | hydrure | mmol | alcoolate | mmol | solvant | temp. | Durée |
|---|---|---|---|---|---|---|---|---|---|
| 1 | Ni(OAc)$_2$<br>Al(Acac)$_3$ | 10<br>10 | LiH | 90 | t-BuOLi | 20 | THF | 63°C | 6 h |
| 2 | Ni(OAc)$_2$<br>Al(Acac)$_3$ | 10<br>20 | LiH | 120 | t-BuOLi | 20 | THF | 63°C | 10 h |
| 3 | Ni(OAc)$_2$<br>Co(OAc)$_2$<br>Al(Acac)$_3$ | 10<br>10<br>10 | LiH | 110 | t-BuOLi | 20 | THF | 63°C | 12 h |
| 4 | Ni(OAc)$_2$<br>Al(Acac)$_3$ | 10<br>10 | NaH | 90 | t-BuOLi | 20 | THF | 63°C | 5 h |
| 5 | NiCl$_2$<br>Al(Acac)$_3$ | 10<br>10 | NaH | 90 | t-BuOLi | 20 | THF | 63°C | 6 h |
| 6 | Ni(Acac)$_2$<br>Al(Acac)$_3$ | 10<br>10 | NaH | 90 | t-BuOLi | 20 | THF | 63°C | 5 h |
| 7 | Ni(Acac)$_2$<br>AlCl$_3$ | 10<br>10 | NaH | 90 | t-BuOLi | 20 | THF | 63°C | 5 h |
| 8 | Ni(Acac)$_2$<br>Al(Acac)$_3$ | 10<br>10 | NaH | 80 | t-BuOLi | 10 | THF | 63°C | 3 h |
| 9 | Ni(Acac)$_2$<br>Al(Acac)$_3$ | 10<br>10 | NaH | 95 | t-BuOLi | 25 | THF | 63°C | 2 h |
| 10 | Ni(OAc)$_2$<br>Al(Acac)$_3$ | 10<br>10 | NaH | 90 | t-BuOLi<br>bipyridine | 20<br>20 | THF | 63°C | 3 h |

**Exemple 11**

Les particules obtenues par le procédé de l'exemple 1 ont été caractérisées par microscopie électronique à transmission, à l'aide d'un microscope Philips CM20/STEM. La caractérisation a montré que la taille des particules ultrafines obtenues est de l'ordre du nanomètre.

Une microanalyse X par dispersion d'énergie, effectuée sur les échantillons Al-Ni a montré qu'il existe une répartition homogène (à l'échelle de 100 nm) des espèces métalliques Al et Ni. Une analyse quantitative basée sur l'exploitation des signaux X émis lors des diffusions inélastiques avec transfert important d'énergie indique que la compositon des éléments métalliques est de 50/50 en nombre d'atomes.

**Exemples 12 à 17**

Les exemples 12, 14 et 16 concernent une réaction d'hydrogénation catalysée par des particules polymétalliques obtenues selon l'exemple 4 ci-dessus.

Les exemples 13, 15 et 17 concernent une réaction d'hydrogénation effectuée dans des conditions strictement identiques à celles des exemples 12, 14 et 16, à l'aide de particules monométalliques de Ni obtenues par Brunet, et al, Journal of Organic Chemistry 1980, vol. 45, pp. 1937-1945.

Dans tous les cas, la réaction d'hydrogénation a été effectuée dans un appareillage classique pour une hydrogénation sous pression atmosphérique. Les conditions opératoires étaient les suivantes :

- substrat à hydrogéner : 20 mmoles
- pression d'hydrogène : 1 bar (= $10^5$ Pa)
- solvant : 15 ml d'éthanol.

L'avancement de la réaction a été déterminé par mesure de l'hydrogène absorbé. Les cinétiques d'absorption d'hydrogène sont représentées sur les figures 3, 4 et 5.

Les courbes 1 et 2 de la figure 3 correspondent respectivement aux exemples 12 et 13.

Les courbes 1 et 2 de la figure 4 correspondent respectivement aux exemples 14 et 15.

Les courbes 1 et 2 de la figure 5 correspondent respectivement aux exemples 16 et 17.

Le rendement en produit hydrogéné formé au cours du temps a été déterminé par dosage chromatographique en phase gazeuse des prélèvements aliquotes. Les rendements sont exprimés en pourcentage molaire.

Le tableau 2 suivant donne les conditions réactionnelles pour les différents exemples.

Tableau 2

| Exemple | | | | | | Résultat | | |
|---------|---|------|-------|------|-----|----------------------|----------------------|-----------------------|
| | S | Cat. | V(ml) | % Ni | PR | $t_{50\%}$ (min) | $t_{90\%}$ (min) | $t_{100\%}$ (min) |
| 12 | Styrène | Ni-Al | 0,5 | 0,625 | éthylbenzène | 4,5 | 17 | 20 |
| 13 | Styrène | Ni | 0,5 | 0,625 | éthylbenzène | 21 | 38 | 45 |
| 14 | cyclooctène | Ni-Al | 4 | 5 | cyclooctane | 24 | 66 | 105 |
| 15 | cyclooctène | Ni | 4 | 5 | cyclooctane | 45 | 123 | 175 |
| 16 | diphénylacétylène | Ni-Al | 1,5 | 1,875 | diphényl éthane | 7 | 15 | 16 |
| 17 | diphénylacétylène | Ni | 3 | 3,75 | diphényl éthane | 14 | 26 | 35 |

- S : substrat à hydrogéner
- V : volume de suspension utilisée comme catalyseur (issue de l'exemple 4 pour les exemples 12, 14 et 16 ; issue du procédé décrit dans Brunet, précité, pour les exemples 13, 15 et 17)
- % Ni : pourcentage molaire de nickel dans le milieu réactionnel
- PR : produit de la réaction
- $t_{50\%}$ : durée exprimée en minutes, nécessaire pour obtenir un rendement molaire de 50%.
- $t_{90\%}$ : durée exprimée en minutes, nécessaire pour obtenir un rendement molaire de 90%.
- $t_{100\%}$ : durée exprimée en minutes, nécessaire pour obtenir un rendement molaire de 100%.

Il apparaît que pour les réactions d'hydrogénation, les durées nécessaires pour obtenir un rendement donné sont fortement réduites, voire approximativement divisées par 2, lorsqu'un catalyseur monométallique est remplacé par un catalyseur polymétallique.

**Exemples 18 à 27**

Les exemples 18, 20, 22, 24 et 26 concernent une réaction de couplage direct de dérivés halogénés aromatiques catalysée des particules polymétalliques obtenues selon l'exemple 10 ci-dessus.

Les exemples 19, 21, 23, 25 et 27 concernent une réaction de couplage direct de dérivés halogénés aromatiques effectuée dans des conditions strictement identiques à celles des exemples 18, 20, 22, 24 et 26, à l'aide de particules monométalliques obtenues selon Lourak, et al, Journal of Organic Chemistry 1989, vol. 54, pp. 4840-4844, c'est-à-dire un système monométallique de nickel.

Le mode opératoire utilisé pour chacun des exemples 18 à 27 est le suivant : on a ajouté goutte à goutte le dérivé halogéné aromatique en solution dans 10 ml de THF à la suspension de particules obtenue dans l'exemple 10 pour les exemples 18, 20, 22, 24 et 26 et à la suspension de particules obtenue selon le procédé Lourak ci-dessus pour les exemples 19, 21, 23, 25 et 27. Les quantités de dérivé halogéné utilisées sont indiquées dans le tableau 3 ci-dessous.

Le rendement en produit de couplage formé au cours du temps a été déterminé par dosage chromatographique en phase gazeuse d'après des prélèvements aliquotes. Les rendements obtenus sont exprimés en pourcentage molaire d'une part par rapport au dérivé halogéné de départ et d'autre part par rapport à la quantité de nickel utilisée.

Tableau 3

| Ex. | ArX | Quantité ARX (mmoles) | Durée de réaction (h) | Produit de réaction | Rt par rapport à AX | Rt par rapport à Ni |
|---|---|---|---|---|---|---|
| 18 | bromobenzène | 40 | 0,75 | biphényle | 85 | 340 |
| 19 | bromobenzène | 20 | 1,75 | biphényle | 80 | 160 |
| 20 | 2-méthyl bromobenzène | 20 | 0,75 | 2,2'-diméthyl biphényle | 85 | 170 |
| 21 | 2-méthyl bromobenzène | 10 | 0,5 | 2,2'-diméthyl biphényle | 90 | 90 |
| 22 | 3-méthyl bromobenzène | 40 | 1,25 | 3,3-diméthyl biphényle | 64 | 256 |
| 23 | 3-méthyl bromobenzène | 20 | 1,5 | 3,3'-diméthyl biphényle | 50 | 100 |
| 24 | 4-méthyl bromobenzène | 40 | 0,5 | 4,4'-diméthyl biphényle | 87 | 348 |
| 25 | 4-méthyl bromobenzène | 40 | 21 | 4,4'-diméthyl biphényle | 71 | 284 |
| 26 | 2-méthyl bromobenzène | 20 | 0,3 | 4,4'-diméthyl biphényle | 99 | 198 |
| 27 | 2-méthyl bromobenzène | 20 | 1 | 4,4'-diméthyl biphényle | 67 | 134 |

Il apparaît que pour les réactions de couplage, les rendements sont augmentés dans des proportions variables suivant les essais, avec des durées de réaction généralement plus courtes.

**Revendications**

1. Procédé de préparation de particules polymétalliques nanométriques par réduction d'un mélange de sels en solution dans un solvant organique à l'aide d'un hydrure de métal alcalin ou alcalino-terreux, à une température inférieure ou égale à la température de reflux du solvant, l'un au moins des sels étant un sel d'un métal ayant un potentiel d'oxydation standard $E°_{Mn+/M}$ à 25°C supérieur à -1,18 V, **caractérisé en ce que** le mélange de sels en solution comprend au moins un sel d'un métal ayant un potentiel d'oxydation standard $E°_{Mn+/M}$ à 25°C inférieur à -1,20 V.

2. Procédé selon la revendication 1, **caractérisé en ce que** l'on ajoute au milieu réactionnel, un alcoolate de métal alcalin, de métal alcalino-terreux ou d'un métal de transition, ou un aminoalcoolate de métal alcalin, de métal alcalino-terreux ou de métal de transition.

**3.** Procédé selon la revendication 1, **caractérisé en ce que** les sels sont choisis dans le groupe constitué par les halogénures, les sels d'acides organiques et les alcoolates.

**4.** Procédé selon la revendication 1, **caractérisé en ce que** le sel d'acide organique est un acétate ou un acétylacétonate.

**5.** Procédé selon la revendication 1, **caractérisé en ce que** le métal du sel de métal ayant un potentiel d'oxydation standard $E^o_{Mn+/M}$ à 25°C inférieur à -1,20 V est choisi dans le groupe constitué par Zr, Ce, Ti, Hf et Al.

**6.** Procédé selon la revendication 1, **caractérisé en ce que** le métal du sel d'un métal ayant un potentiel d'oxydation standard $E^o_{Mn+/M}$ à 25°C supérieur à -1,18 V est choisi dans le groupe constitué par Ni, Co, Fe, Cu, Zn, Cd, Cr, Mn, Pd, Ag, Pt, Au, Bi, Sb.

**7.** Procédé selon la revendication 1, **caractérisé en ce que** l'hydrure de métal alcalin est choisi dans le groupe constitué par l'hydrure de sodium, l'hydrure de lithium et l'hydrure de potassium.

**8.** Procédé selon la revendication 1, **caractérisé en ce que** l'hydrure de métal alcalino-terreux est l'hydrure de calcium ou l'hydrure de magnésium.

**9.** Procédé selon la revendication 2, caractérisé en ce l'alcoolate est choisi parmi le tertiobutylate de sodium, le tertiobutylate de lithium et l'isopropylate d'aluminium, et l'aminoalcoolat est le N,N-diméthylaminoéthanolate de sodium.

**10.** Procédé selon la revendication 9, **caractérisé en ce que** l'on forme l'alcoolate in situ par action de l'hydrure sur un alcool aliphatique ou aromatique.

**11.** Procédé selon la revendication 10, **caractérisé en ce que** l'alcool est choisi dans le groupe constitué par le tertiobutanol, le méthyl-2-pentanol-2, le phénol, le monoéthyl étherdiéthylène glycol, le néopentanol, le cyclohexanol, l'éthanol, l'isopropanol.

**12.** Procédé selon la revendication 1, **caractérisé en ce que** la réaction de réduction du mélange de sels est effectuée à une température comprise dans l'intervalle limité par la température de solidification et par la température de reflux du solvant utilisé.

**13.** Procédé selon la revendication 1, **caractérisé en ce que** l'on ajoute au milieu réactionnel un composé comprenant un ligand azoté ou phosphoré.

**14.** Procédé selon la revendication 13, **caractérisé en ce que** le composé comprenant un ligand azoté est choisi dans le groupe constitué par les bipyridines et les terpyridines.

**15.** Procédé selon la revendication 13, **caractérisé en ce que** le composé comprenant un ligand phosphoré est choisi dans le groupe constitué par la triphénylphosphine, le 1,2-bis(diphénylphosphino)éthane, les alkylphosphines et les arylphosphines.

**16.** Matériau constitué par des particules nanométriques polymétalliques ayant une dimension moyenne de cristallites inférieure à 4 nm et une association intime des divers métaux présents à l'intérieur des grains, l'un au moins des métaux constituant lesdites particules étant un métal ayant un potentiel d'oxydation standard $E^o_{Mn+/M}$ à 25°C supérieur à -1,18 V et l'un au moins des métaux constituant les particules est un métal ayant un potentiel d'oxydation standard $E^o_{Mn+/M}$ à 25°C inférieur à -1,20 V.

**17.** Matériau selon la revendication 16, **caractérisé en ce que** le métal ayant un potentiel d'oxydation standard $E^o_{Mn+/M}$ à 25°C inférieur à -1,20 V est choisi dans le groupe constitué par Zr Ce, Ti, Hf et Al.

**18.** Matériau selon la revendication 16, **caractérisé en ce que** le métal ayant un potentiel d'oxydation standard $E^o_{Mn+/M}$ à 25°C supérieur à -1,18 V est choisi dans le groupe constitué par Ni, Co, Fe, Cu, Zn, Cd, Cr, Mn, Pd, Ag, Pt, Au, Bi, Sb.

**19.** Procédé d'hydrogénation d'oléfines, **caractérisé en ce qu'**il est mis en oeuvre en présence d'un matériau obtenu par le procédé selon l'une des revendications 1 à 12.

20. Procédé de couplage de dérivés halogénés aromatiques, **caractérisé en ce qu'**il est mis en oeuvre en présence d'un matériau obtenu par le procédé de l'une des revendication 13 à 15.

**Claims**

1. Process for the preparation of nanometric polymetallic particles by reduction of a mixture of salts in solution in an organic solvent using an alkali metal or alkaline earth metal hydride at a temperature of less than or equal to the reflux temperature of the solvent, wherein at least one salt of a metal has a standard oxidation potential $E^o_{Mn+/M}$ at 25°C higher than -1.18 V, **characterized in that** the mixture of salts in solution comprises at least one salt of a metal having a standard oxidation potential $E^o_{Mn+/M}$ at 25°C of less than -1.20 V.

2. Process according to Claim 1, **characterized in that** an alkali metal, alkaline earth metal or transition metal alkoxide or an alkali metal, alkaline earth metal or transition metal aminoalkoxide is added to the reaction medium.

3. Process according to Claim 1, **characterized in that** the salts are chosen from the group consisting of halides, organic acid salts and alkoxides.

4. Process according to Claim 1, **characterized in that** the organic acid salt is an acetate or an acetylacetonate.

5. Process according to Claim 1, **characterized in that** the metal of the salt of a metal having a standard oxidation potential $E^o_{Mn+/M}$ at 25°C of less than -1.20 V is chosen from the group consisting of Zr, Ce, Ti, Hf and Al.

6. Process according to Claim 1, **characterized in that** the metal of the salt of a metal having a standard oxidation potential $E^o_{Mn+/M}$ at 25°C of greater than -1.18 V is chosen from the group consisting of Ni, Co, Fe, Cu, Zn, Cd, Cr, Mn, Pd, Ag, Pt, Au, Bi and Sb.

7. Process according to Claim 1, **characterized in that** the alkali metal hydride is chosen from the group consisting of sodium hydride, lithium hydride and potassium hydride.

8. Process according to Claim 1, **characterized in that** the alkaline earth metal hydride is calcium hydride or magnesium hydride.

9. Process according to Claim 2, **characterized in that** the alkoxide is chosen from sodium tert-butoxide, lithium tert-butoxide and aluminum isopropoxide and the aminoalkoxide is sodium N,N-dimethylaminoethoxide.

10. Process according to Claim 9, **characterized in that** the alkoxide is formed in situ by the action of the hydride on an aliphatic or aromatic alcohol.

11. Process according to Claim 10, **characterized in that** the alcohol is chosen from the group consisting of tert-butanol, 2-methyl-2-pentanol, phenol, diethylene glycol monoethyl ether, neopentanol, cyclohexanol, ethanol and isopropanol.

12. Process according to Claim 1, **characterized in that** the reaction in which the mixture of salts is reduced is carried out at a temperature within the range limited by the solidifying temperature and by the reflux temperature of the solvent used.

13. Process according to Claim 1, **characterized in that** a compound comprising a nitrogen-comprising or phosphorus-comprising ligand is added to the reaction medium.

14. Process according to Claim 13, **characterized in that** the compound comprising a nitrogen-comprising ligand is chosen from the group consisting of bipyridines and terpyridines.

15. Process according to Claim 13, **characterized in that** the compound comprising a phosphorus-comprising ligand is chosen from the group consisting of triphenylphosphine, 1,2-bis(diphenylphosphino)ethane, alkylphosphines and arylphosphines.

16. Material composed of nanometric polymetallic particles having a mean crystallite dimension of less than 4 nm and an intimate association of the various metals present inside the grains, at least one of the metals constituting said particles being a metal having a standard oxidation potential $E^o_{Mn+/M}$ at 25°C of greater than -1.18 V and at least one of the metals constituting the particles is a metal having a standard oxidation potential $E^o_{Mn+/M}$ at 25°C of less than -1.20 V.

17. Material according to Claim 16, **characterized in that** the metal having a standard oxidation potential $E^o_{Mn+/M}$ at 25°C of less than -1.20 V is chosen from the group consisting of V, Zr, Ce, Ti, Hf and Al.

18. Material according to Claim 16, **characterized in that** the metal having a standard oxidation potential $E^o_{Mn+/M}$ at 25°C of greater than -1.18 V is chosen from the group consisting of Ni, Co, Fe, Cu, Zn, Cd, Cr, Mn, Pd, Ag, Pt, Au, Bi and Sb.

19. Process for hydrogenating olefins, **characterized in that** it is carried out in the presence of a material obtained by the process according to one of Claims 1 to 12.

20. Process for coupling halogenated aromatic derivatives, **characterized in that** it is carried out in the presence of a material obtained by the process of one of Claims 13 to 15.

**Patentansprüche**

1. Verfahren zur Herstellung von Mehrmetallteilchen im Nanometergrößenbereich durch Reduktion einer Salzmischung in Lösung in einem organischen Lösungsmittel mithilfe eines Alkalimetall- oder Erdalkalimetallhydrids bei einer Temperatur ≤ der Rückflusstemperatur des Lösungsmittels, wobei zumindest eines der Salze ein Salz eines Metalls mit einem Standard-Oxidationspotential $E^o$ $M^{n+}/M$ bei 25 °C über -1,18 V ist, **dadurch gekennzeichnet, dass** die Salzmischung in Lösung zumindest ein Salz eines Metalls mit einem Standard-Oxidationspotential $E^o$ $M^{n+}/M$ bei 25 °C unter -1,20 V umfasst.

2. Verfahren nach Anspruch 1, **dadurch gekennzeichnet, dass** dem Reaktionsmedium ein Alkalimetall-, Erdalkalimetall- oder Übergangsmetallalkoholat oder ein Alkalimetall-, Erdalkalimetall- oder Übergangsmetallaminoalkoholat zugegeben wird.

3. Verfahren nach Anspruch 1, **dadurch gekennzeichnet, dass** die Salze aus der Gruppe ausgewählt sind, die aus den Halogeniden, den Salzen organischer Säuren und den Alkoholaten besteht.

4. Verfahren nach Anspruch 1, **dadurch gekennzeichnet, dass** das organische Säuresalz ein Acetat oder ein Acetylacetonat ist.

5. Verfahren nach Anspruch 1, **dadurch gekennzeichnet, dass** das Metall des Metallsalzes mit einem Standard-Oxidationspotential $E^o$ $M^{n+}/M$ bei 25 °C unter -1,20 V aus der aus Zr, Ce, Ti, Hf und Al bestehenden Gruppe ausgewählt ist.

6. Verfahren nach Anspruch 1, **dadurch gekennzeichnet, dass** das Metall des Metallsalzes mit einem Standard-Oxidationspotential $E°$ $M^{n+}/M$ bei 25 °C über -1,18 V aus der aus Ni, Co, Fe, Cu, Zn, Cd, Cr, Mn, Pd, Ag, Pt, Au, Bi, Sb bestehenden Gruppe ausgewählt ist.

7. Verfahren nach Anspruch 1, **dadurch gekennzeichnet, dass** das Alkalimetallhydrid aus der aus Natriumhydrid, Lithiumhydrid und Kaliumhydrid bestehenden Gruppe ausgewählt ist.

8. Verfahren nach Anspruch 1, **dadurch gekennzeichnet, dass** das Erdalkalimetallhydrid Kalziumhydrid oder Magnesiumhydrid ist.

9. Verfahren nach Anspruch 2, **dadurch gekennzeichnet, dass** das Alkoholat aus Natrium-t-butylat, Lithium-t-butylat und Aluminiumisopropylat ausgewählt ist und das Aminoalkoholat Natrium-N,N-dimethylaminoethanolat ist.

10. Verfahren nach Anspruch 9, **dadurch gekennzeichnet, dass** das Alkoholat in situ durch Einwirken des Hydrids auf einen aliphatischen oder aromatischen Alkohol gebildet wird.

**11.** Verfahren nach Anspruch 10, **dadurch gekennzeichnet, dass** der Alkohol aus der aus t-Butanol, Methyl-2-pentanol-2, Phenol, Monoethyletherdiethylenglykol, Neopentanol, Cyclohexanol, Ethanol und Isopropanol bestehenden Gruppe ausgewählt ist.

**12.** Verfahren nach Anspruch 1, **dadurch gekennzeichnet, dass** die Reduktionsreaktion der Salzmischung bei einer Temperatur im Bereich zwischen der Verfestigungstemperatur und der Rückflusstemperatur des eingesetzten Lösungsmittels durchgeführt wird.

**13.** Verfahren nach Anspruch 1, **dadurch gekennzeichnet, dass** dem Reaktionsmedium eine Verbindung zugegeben wird, die einen Stickstoff- oder Phosphorliganden enthält.

**14.** Verfahren nach Anspruch 13, **dadurch gekennzeichnet, dass** die einen Stickstoffliganden enthaltende Verbindung aus der aus Bipyridinen und Terpyridinen bestehenden Gruppe ausgewählt ist.

**15.** Verfahren nach Anspruch 13, **dadurch gekennzeichnet, dass** die einen Phosphorliganden enthaltende Verbindung aus der aus Triphenylphosphin, 1,2-Bis(diphenylphosphino)ethan, Alkylphosphinen und Arylphosphinen bestehenden Gruppe ausgewählt ist.

**16.** Material, bestehend aus Mehrmetallteilchen im Nanometergrößenbereich mit einer mittleren Kristallitgröße unter 4 nm und einer engen Assoziation der verschiedenen im Inneren der Körner vorhandenen Metalle, wobei zumindest eines der Metalle, aus denen die Teilchen bestehen, ein Metall mit einem Standard-Oxidationspotential $E^o$ $M^{n+}/M$ bei 25 °C über -1,18 V ist und zumindest eines der Metalle, aus denen die Teilchen bestehen, ein Metall mit einem Standard-Oxidationspotential $E^o$ $M^{n+}/M$ bei 25 °C unter -1,20V ist.

**17.** Material nach Anspruch 16, **dadurch gekennzeichnet, dass** das Metall mit einem Standard-Oxidationspotential $E^o$ $M^{n+}/M$ bei 25 °C unter -1,20 V aus der aus Zr, Ce, Ti, Hf und Al bestehenden Gruppe ausgewählt ist.

**18.** Material nach Anspruch 16, **dadurch gekennzeichnet, dass** das Metall mit einem Standard-Oxidationspotential $E^o$ $M^{n+}/M$ bei 25 °C über -1,18 V aus der aus Ni, Co, Fe, Cu, Zn, Cd, Cr, Mn, Pd, Ag, Pt, Au, Bi und Sb bestehenden Gruppe ausgewählt ist.

**19.** Verfahren zur Hydrierung von Olefinen, **dadurch gekennzeichnet, dass** es in Gegenwart eines nach dem Verfahren nach einem der Ansprüche 1 bis 12 erhaltenen Materials durchgeführt wird.

**20.** Verfahren zur Kopplung von aromatischen halogenierten Derivaten, **dadurch gekennzeichnet, dass** es in Gegenwart eines nach dem Verfahren nach einem der Ansprüche 13 bis 15 erhaltenen Materials durchgeführt wird.

Fig. 1

Fig. 2

Fig. 3

Fig. 4

EP 1 019 191 B1

16

Fig.5